(19) 

Europäisches
Patentamt
European
Patent Office
Office européen
des brevets

(11) **EP 4 060 383 A1**

(12) **EUROPEAN PATENT APPLICATION**

(43) Date of publication:
**21.09.2022 Bulletin 2022/38**

(21) Application number: **21315044.4**

(22) Date of filing: **19.03.2021**

(51) International Patent Classification (IPC):
**G01S 15/89** (2006.01)    **G01S 7/52** (2006.01)
**A61B 8/08** (2006.01)

(52) Cooperative Patent Classification (CPC):
**G01S 15/8993; A61B 5/0035; A61B 5/0071;
G01S 7/52071;** A61B 6/032; A61B 6/4417;
A61B 6/5247; A61B 8/4416; A61B 8/485;
A61B 8/5261

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **SuperSonic Imagine
13857 Aix-en-Provence Cedex 3 (FR)**

(72) Inventor: **Bo, Zhang
13480 Cabries (FR)**

(74) Representative: **Betten & Resch
Patent- und Rechtsanwälte PartGmbB
Maximiliansplatz 14
80333 München (DE)**

(54) **METHOD AND SYSTEM FOR PROCESSING MULTI-MODALITY AND/OR MULTI-SOURCE DATA OF A MEDIUM**

(57)    The invention relates to for method for processing multi-modality and/or multi-source data of a medium, wherein said method may be implemented by a processing system, the method comprising the following steps: an optical determination step in which for at least one of a plurality of volume units of the medium an optical property is determined based on the data of a modality and/or source,

a fluorescence determination step in which for at least one of the volume units a fluorescence property is determined based on the data of a second modality and/or source, and
a rendering step in which a data representation of the medium is rendered based on the determined optical and fluorescence properties of the volume units. The invention also relates to a corresponding processing system.

**Fig. 1**

EP 4 060 383 A1

**Description**

FIELD OF THE DISCLOSURE

The present disclosure relates to a method and system for processing multi-modality and/or multi-source data of a medium. In particular, the present disclosure concerns image processing methods and systems implementing said methods, in particular for medical imaging.

BACKGROUND OF THE DISCLOSURE

Examination, in particular for example medical examination, is often assisted by computer implemented imaging methods, for example ultrasound imaging. For this purpose, examination data from an examined medium (for example stones, animals, human body or part of it) is acquired and processed, in order to present it to the examining user or to another user such as a doctor.

For example, ultrasound imaging consists of an insonification of a medium with one or several ultrasound pulses (or waves) which are transmitted by a transducer. In response to the echoes of these pulses ultrasound signal data are acquired, as example by using the same transducer. Ultrasound imaging may have different modalities (or modes), for example B-mode (brightness mode) and ShearWave® (SWE, shear wave elastography), which may be provided by the same ultrasound imaging system.

The interpretation of such images, or more generally speaking of any examination data, requires though high expert knowledge. This is in particular the case for three-dimensional image data, for example 3D ultrasound images.

The examination data may become even more complex, in case data of different modalities and/or different sources are combined, for example into the same 3D (three dimensions) representation shown to the user. Such different modalities may comprise data obtained by the same system in different modes, for example B-mode image data and SWE image data obtained by an ultrasound system. In other words, data of different modalities may comprise data of the same source (for example ultrasound) but of different modalities (for example different ultrasound acquisition modes).

The different sources may also comprise data obtained by different systems, for example by an ultrasound system and another imaging system, for example a medical imaging system like computer tomography, magnetic resonance or positron emission tomography.

As a result, the visual representation of combined multi-modality and/or multi-source data (for example a rendered 3D image) may provide an information overload to the user, conducting to less clarity and potentially source of misinterpretation in complex cases such that more relevant information may be disguised by other less relevant one.

There exist different known techniques for rendering images, in particular 3D images or images with depth information. For example, for medical image visualization, different volume-rendering algorithms have been evolved, as described in Zhang Q, Eagleson R, Peters TM. Volume visualization: a technical overview with a focus on medical applications. J Digit Imaging. 2011 Aug;24(4):640-64. doi: 10.1007/s10278-010-9321-6. PMID: 20714917; PMCID: PMC3138940.

In particular, multiplanar reformation (MPR) is a known image processing technique, which extracts two-dimensional (2D) slices from a 3D volume using arbitrarily positioned orthogonal or oblique planes. MPR is a standard solution on commercial ultrasound systems with 3D acquisition.

Another example is surface rendering (SR) which requires the object surface to be explicitly or implicitly modelled. The surface is then shaded and rendered, for example by the method of ray-tracing. This technique is vastly used on video games and digital films. But it is less adapted to medical ultrasound context, as surface modelling requires automatic segmentation of a priori unknown number of unknown structures, which is practically hardly robust, and requires additional computational cost.

Still another example is volume rendering (VR) which displays the entire 3D data as a 2D (two dimensions) image without computing any intermediate geometrical representation.

However, these techniques do not solve or reduce the problems stated above.

Moreover, medical examination (for example using an ultrasound imaging method) often requires real time or quasi real time monitoring, in particular for medical intervention guided by for example ultrasound imaging. For this reason, both the increased calculation costs of the known rendering methods and the potential information overload of the user may be prejudicial with regard to the real-time requirement.

SUMMARY OF THE DISCLOSURE

Currently, it remains desirable to overcome the aforementioned problems and in particular to provide a method and system for processing multi-modality and/or multi-source data of a volumetric medium which facilitates the visualization, understanding and interpretation of the multi-modality and/or multi-source data. Moreover, the method and system desirably provide a combined or merged representation of said multi-modality and/or multi-source data in a respective data representation, for example in a 3D image or 2D image with depth information.

Therefore, according to the embodiments of the present disclosure, a method for processing multi-modality and/or multi-source data of a (for example volumetric) medium is provided. Said method may be implemented by a processing system. The method comprises the following steps:

- an optical determination step in which for at least one of a plurality of volume units of the medium an optical property is determined based on the data of a first modality and/or source,
- a fluorescence determination step in which for at least one of the volume units a fluorescence property is determined based on the data of a second modality and/or source, and
- a rendering step in which a data representation of the volumetric medium is rendered based on the determined optical and fluorescence properties of the volume units.

By providing such a method, the visualization, understanding and/or interpretation of data (for example medical imaging data) from different modalities and/or sources can be facilitated, becoming thus more intuitive, both for expert users (for example medical professionals) and for non-expert users (for example non-professionals such as patients or non-trained professionals) in an optically plausible way.

The present disclosure may adapt a conventional rendering algorithm by combining it with a modelling of multi-color fluorescence mechanism. In this way data volume units having different modalities and/or sources may be combined and be interpretable in a unified rendering scene.

Accordingly, the proposed technique may enable multiple imaging modes and/or imaging sources (for example, 3D B-mode as a first modality and 3D SWE mode as a second modality) to be rendered by different customizable fluorescence colors such that they are visualizable in a unified scene. In this example, medium echogenicity information from B-mode imaging and tissue elasticity information from SWE may be simultaneously rendered.

In other words, medium properties can be combined in the same visualizable scene. This may lead to visual clues for user guidance, for monitoring of pathology or of treatment, and also lead to easier user and patient understanding and interpreting of multi-modality and/or multi-source data.

As a further consequence, the resulting unified scene may offer an improved basis for any machine learning or other AI-related applications. For example, the scene may be in the form of a 2D or 3D image with a predefined size which can be processed by a CNN (convolutional neural network) or another machine learning algorithm which expects input data of a predefined size and/or format. As a consequence, the 2D or 3D image can be used as a single and standardized input for the CNN (or any other machine learning algorithm) without requiring any further pre-processing. In contrast, the original multi-modality and/or multi-source data may have different or varying resolutions which would require for each case a specific pre-processing.

Moreover, due to the determination of optical properties and fluorescent properties, the resulting rendered representations may be enhanced in contrast and may comprise less noise, in comparison to the original multi-modality and/or multi-source data. This circumstance may not only help a human being to correctly interpret the rendered representation. It may also ameliorate the results of any classification and/or regressions tasks carried out by a machine learning algorithm.

For example, the rendered data representation may be such that a more realistic weighting of the original data is provided to the AI-based algorithm. In other words, the (AI-based) algorithm may be more sensitive to the more weighted, i.e. more relevant data. In one example, data of a SWE modality may obtain an increased weighting or attention (i.e. may be highlighted and/or colored) through the fluorescence determination step. In other words, said data (for example marking a lesion in the medium) may also be processed by the AI-based algorithm with an increased attention.

The steps of the present disclosure may also be carried out or at least assisted by one or several machine learning algorithms or any other AI (artificial intelligence) based algorithms.

A further advantage of the proposed method is the possible reduction of computational costs. For example, the proposed method enables multi-mode 3D image blending without object or surface segmentation. Furthermore. the computational cost is advantageously independent of number of objects in the scene, and therefore it is not necessary to make multiple renderings for multiple objects. The computational cost may only depend on the number of managed volume units. Moreover, the determination of fluorescent properties relies on physics principle of fluorescence and not on arbitrary shading. This facilitates practical parameters optimization by providing more physical intuition and as such being more self-explanatory.

The method may further comprise a step of segmenting the medium into a plurality of volume units. Said volume units may anyway already be provided by the multi-modality and/or multi-source data, for example in the form of voxels in three-dimensional multi-modality and/or multi-source image data. It is further possible that there are also volume units outside the medium. At least one of these volume units outside the medium may be occupied by another means which is considered in the rendered data representation, for example an intervention device. For example, the voxels representing sampled locations of the medium and/or the body of an intervention device may be arranged in a coordinate system, for instance a cartesian or polar system or any other predefined system.

In one example, the data of modality and/or source may comprise 3D image information of the medium. Said information may be acquired on a voxel by voxel mode, in accordance to a predefined coordinate system such as a cartesian or polar system or other predefined system. Such 3D image information may be acquired for example using an ultrasound matrix probe, i.e. having a plurality of transducers arranged in a matrix form or a probe merging several transducers types.

3D image information may also be obtained from stacking 2D image slices. This may be achieved from a mechanical 1D linear ultrasound probe. The probe may be mechanically rotated in a given direction, and at different angles the 1D probe acquires a 2D image slice.

The at least one volume unit for which an optical property is determined, may be the same or different volume as the volume unit for which a fluorescence property is determined. Accordingly, one specific volume unit may have a determined optical property and/or a determined fluorescent property, or none of both. Moreover, it is possible that for at least one volume unit none of the optical property and the fluorescence property is determined.

In one exemplary embodiment, for several volume units, an optical property is determined and/or for several volume units a fluorescent property is determined. In another example, for each of the plurality of volume units an optical property is determined and/or each of the plurality of volume units a fluorescent property is determined.

The rendering step may be understood as a computation step or calculation step. It may also comprise a graphical rendering step and/or a 3D rendering step.

The data representation of the volumetric medium may comprise a visual or graphical representation with a plurality of depth levels.

In one example, the data representation, in particular said visual representation may comprise or may be in the form of one or several images, for example a two-dimensional (2D) or a three-dimensional (3D) image.

The optical property may be understood as a passive optical property.

The fluorescent property may be understood as the property of emitting (secondary) light source. Accordingly, the difference between "optical property" and "fluorescence property" may be that the former is passive, and the latter is active. "Passive" may mean that they are no lighting sources, and "active" may means that they can emit light.

It is also possible that the optical determination step is not only carried out for the data of one first modality and/or source but that the optical property is determined based on the data of at different modalities (for example B-mode and SWE) and/or sources (for example ultrasound and CT).

In the same way, it is also possible that in the fluorescence determination step the fluorescence property is determined based on the data of at different modalities and/or sources. For example, for each modality and/or source a different fluorescence color may be determined (for example red for SWE and blue for marking a recognized intervention device (such as needle, marker or a region of interest in the medium), in order to facilitate distinguishing the different modalities and/or sources.

In one example the optical property may comprise or may be a light absorption rate of a volume unit.

In a further example, the optical property may be determined according to a (for example predefined) medium-light interaction mapping applied to the data of the first modality and/or source of the at least one volume unit. Accordingly, the data of the first modality and/or source may be used as an input of the medium-light interaction mapping. The optical property may be the output of the medium-light interaction mapping.

The medium-light interaction mapping may determine at least one of a reflectivity, directivity, and absorption rates as a function of the data of the of the first modality and/or source.

This mapping may be or may comprise a predefined mapping function and/or a predefined mapping rule. For example, the mapping may determine (based on a mapping rule) which kind of modality and/or source is used to determine the optical properties. For instance, optical properties may be determined based on B-mode data (being the first modality and/or source in this example). As a further example, the mapping may determine (based on a mapping rule and/or function) values or value ranges of a parameter of optical properties (for example reflectivity, directivity and absorption rate) as a function of values or value ranges of parameters of the first modality and/or source (for example the brightness values in a B-mode data).

The fluorescence property may be determined according to a (for example predefined) light emission and/or absorption mapping applied to the data of the second modality and/or source of the at least one volume unit. Said mapping may for example be a light emission mapping or a light emission and absorption mapping. Accordingly, said mapping may define a light emission rate for the at least one volume unit.

Accordingly, also this light emission and/or absorption mapping may be or may comprise a predefined mapping function and/or a predefined mapping rule. For example, the mapping may determine (based on a mapping rule) which kind of modality and/or source is used to determine the fluorescence properties. For instance, fluorescence properties may be determined based on SWE data (being the second modality and/or source in this example). As a further example, the mapping may determine (based on a mapping rule and/or function) values or value ranges of a parameter of fluorescence properties (for example a light emission rate, light absorption rate and/or color) as a function of values or value ranges of parameters of the second modality and/or source (for example the brightness/color values in SWE data).

According to another example, a mapping according to the present disclosure may map an optical property and/or a fluorescence property for a given voxel values. It is also possible that the mapping determines a map. Said map may provide a data structure corresponding to the data structure of the source and/or modality data. For example, the map may be of the same size as an image forming the source and/or modality data. The map may hence store the optical/fluorescence property at each pixel/voxel.

The fluorescence property may define a secondary light source formed by a volume unit. In other words, the volume unit may emit secondary light according to the fluorescence property. Accordingly, said secondary light may be determined based on primary light absorbed in a first spectrum and (re-)emitted as secondary light in a second spectrum by the volume unit.

In the optical determination step for at least one of the volume units an optical property may be determined based on the data of a third modality and/or source according to a second medium-light interaction mapping being different to the first medium-light interaction mapping.

In the fluorescence determination step for at least one of the volume units a fluorescence property may be determined based on the data of a fourth modality and/or source according to a second light emission and/or absorption mapping being different to the first light emission and/or absorption mapping.

In other words, data of different modalities and/or sources may be processed according to different mappings, for example to obtain different fluorescence and/or optical properties for each data modality and/or source, respectively. In this way, different data modalities and/or sources may be distinguished more easily and more intuitively, for instance by a human being or any AI tools. For example,

The light emission and absorption mapping may also be customizable by a user of the processing system. In this way, the user may freely choose the way a specific data modality and/or source may be for example highlighted and/or colored.

The fluorescence determination step and the optical determination step may be carried out simultaneously and/or in parallel. In this way, the total processing time of the method may be reduced.

The rendering step may be carried out after the fluorescence determination step and/or the optical determination step. In this way, the rendering step may use the data determined in the fluorescence determination step and the optical determination step. For example, the determined optical and fluorescence properties may be stored in a data storage (for example a local or cloud data storage). The rendering step may be carried out one or several times based on the stored properties. In this way, it is possible to store the determination in for instance a data model like a 3D-model and perform renderings of different data representations at any later time point.

Alternatively, the rendering step may be carried out simultaneously with (i.e. at the same time as) the fluorescence determination step and/or the optical determination step. For example, in case of using ray-tracing in the rendering step, it is possible to determine for each ray (for example corresponding to a pixel in an 2D image forming the data representation) the optical and fluorescence properties of the volume units met by the said ray.

The data of the first modality and/or source may comprise reflectivity information from B-mode (brightness mode) ultrasound imaging. For example, said first modality may be used to obtain a background for the rendered data representation, for example a background of a 2D or 3D image forming the rendered data representation.

The data of the second modality and/or source may comprise tissue elasticity information from shear wave elastography ultrasound imaging. For example, the second modality may be used to highlight certain areas in the rendered data representation.

According to a further embodiment, the data of the second or a fourth modality and/or source may comprise intervention device information. Said intervention device information may indicate the position and/or orientation of an intervention device (for example its 6D-pose) placed in the medium and/or predetermined information of the shape of the intervention device.

In one example, the intervention device information may comprise coordinates indicating the position and/or orientation of the device in a coordinate system of the medium. Those data may or may not be displayed inside the unified scene, either on same display or may be displayed on another display. Accordingly, the intervention device information does not necessarily comprise image data of the device, but they may do so.

Accordingly, it is possible to include data of a modality and/or source that is not in form of an image. As mentioned above, there may not be determined image information of an interventional device, but only coordinates of it. A fluorescence property may in this case be associated to these coordinates and they may be rendered inside the unified scene comprising the medium and the intervention device.

For example, given a needle as an exemplary intervention device, there are several possibilities of rendering it:

The points on the needle may be indicated voxel by voxel in a volume under the same coordinate system of the B mode image for example. Then the needle source may be another image source aligned with the B mode image and may be treated in the same way as data of any other modality and/or source (for instance SWE) but for instance with a different fluorescence color.

Alternatively, the points on the needle may be represented as a set of coordinates in a coordinate system of a given image, for example B mode image. Then a fluorescence color may be associated to these coordinates. In this way, the needle points are represented by the same coordinate system of the B mode image. According to this alternative, no additional needle image is used.

In still a further alternative, the points on the needle may be represented as a set of coordinates in a global reference coordinate system. For example, a transducer or transmitter of the intervention device (such as a tip of a needle) may send signals to a signal capturer of the system to inform its location. The scanning probe may also send signal to the same capturer to inform its location. Then, this reference coordinate system may be shared by some or all sources. In this way, the rendering scene may be built from this global coordinate system.

In still a further alternative, the intervention device is handled like the medium in the steps of the method of the present disclosure, i.e. as if it was a part of the medium. In other words, there may not be any difference in the disclosed method as applied to the intervention device and the medium. In particular, the same data modalities and/or sources may be used, and the data may be processed in one common way without distinguishing between intervention device and medium.

In the fluorescence determination step for at least one volume unit a fluorescence property may be determined based on the intervention device information. This determination may be done by identifying those volume units of the medium which are occupied by the intervention device (i.e. where the intervention device is located), in particular according to its position and/or orientation and its shape.

Accordingly, in the fluorescence determination step those volume units of the medium, which are occupied by the intervention device, may be colored according to the fluorescence properties determined for the detected intervention device. In this way, in a visual representation for example an intervention device in the medium may be visualized, as described in more detail below.

In the rendering step at least a portion of the data representation may be rendered based on intervention device information. Said intervention device information may indicate the position and/or orientation of an intervention device placed in the medium and predetermined information of the shape of the intervention device.

The position and/or orientation of the intervention device may be determined by scanning the intervention device using scanning waves which are configured based on predetermined information of the intervention device. The predetermined information of the shape of the intervention device may comprise 3D surface information of the intervention device. It may also comprise a set of images (for example 2D or 3D) from different perspectives of the intervention device. Said information may be determined in prior scanning step (for example carried out before the optical determination step and/or the fluorescence determination step) or may be obtained from a data storage, for example a local data storage of the processing system or a remote data storage, for example a cloud system. The data storage may also store intervention device information of different intervention devices. Moreover, it may store further information for an intervention device, for example technical characteristics (such as its material, its spatial dimensions, its weight) and/or manufacturer information (such as a product ID, a manufacturer ID).

For example, B-mode data or any other scanning data acquired in a scanning step may be used to determine a product ID of the intervention device. Based on this product ID, the correct information regarding the shape (potentially 3D shape) of the intervention device may be obtained from the data storage

As a consequence of using the intervention device information in the fluorescence determination step and/or the rendering step, it becomes advantageously possible to visualize both an intervention device (for example a biopsy needle) and the medium addressed by the biopsy (for example anatomical structures).

Furthermore, it is possible to determine where the intervention device is predicted to move to in the medium (for example its moving direction and/or a moving path). Said prediction may be made by a respectively trained (AI-based) algorithm.

The rendering step may comprise a ray-tracing rendering step in which the data representation is rendered according to a ray tracing volume rendering method. For example, the fluorescence determination step and the optical determination step may be each carried out volume unit per volume unit. Then in the rendering step for each pixel of the rendered data representation (for example a 2D or 3D image) a ray is determined and the pixel/voxel color is calculated as a function of the volume units (for example voxels) met by the ray.

However, the optical and fluorescence properties do not need to be determined before the rendering step. It is also possible that the fluorescence determination step and the optical determination step are carried out at the same time as the rendering step. For example, the determination may be a predefined mapping rule between a value of a volume unit (for example a voxel value) and an optical and/or fluorescence property. In such a case their determination may be done at the same time as the rendering itself.

The ray-tracing rendering step may comprise at least one of the sub-steps of:

- positioning a virtual light source in a predefined geometric relation to the medium (for example inside the medium, close or adjacent to the medium, or distanced to the medium),

- defining a depth of field and/or an aperture shape,
- positioning a virtual view plane in a predefined geometric relation to the medium, wherein the data representation is rendered as a function of the virtual view plane. The location of the virtual view plane may be defined as a function of the real distance of the user from the medium, such that it corresponds to the latter real distance. This might be useful when using augmented reality lenses to fit the visual representation into the region where the real medium is located.

The multi-modality and/or multi-source data may comprise image data and/or 3D information data of the medium. A volume unit may comprise at least one voxel. A voxel may represent a value on a regular grid in a 3D space which may form a cartesian coordinate system (for instance when the data of the modality and/or source are acquired by a probe having a matrix-array of transducers). Said 3D space may accordingly include the medium and optionally one or several intervention devices. However, the voxels may also be arranged according to a polar coordinate system (in particular when the data of the modality and/or source are acquired by a probe having a convex array including a plurality of transducers aligned along a curved line.

In general, in case the modalities and/or sources used in the method of the present disclosure have different voxel resolutions and/or different coordinate systems, they may be harmonized to a reference system (comprising a reference resolution of volume units and a reference coordinate system). This allows to fuse their data in the optical and fluorescence determination steps and the rendering step. For example, the data of the modality and/or source of highest resolution may be used as a reference and the data of other modalities and/or sources may be interpolated, respectively. Alternatively, the data of the modality and/or source of lowest resolution may be used as a reference and the data of other modalities and/or sources may be down-sampled, respectively. However, it is not mandatory to (beforehand) harmonize different coordinate systems and/or different resolutions of modalities and/or sources. According to the present disclosure they may also be merged (and thus harmonized) in the rendering step.

For example, different resolutions of data may be handled by interpolation when each ray at the rendering step is computed according to a ray-tracing method. In fact, during the rendering, each ray may be discretized into a sequence of points. The location of these "ray points" are thus known inside each data volume. To sample the data values at these "ray points", data of a modality and/or source from its original resolution may be interpolated. Once the values are sampled, the optical/fluorescence property is determined from a value-property mapping for example. It is not necessary to systematically unify data resolutions of different modalities and/or sources beforehand. The advantage of such a technique is that for example high-resolution data do not need to be degraded because of the presence of another low-resolution source. Likewise, low-resolution data do not need to be up-sampled due the presence of another high-resolution source. Thus the computational costs of the method can be reduced.

For different coordinate systems, the processing may be likewise. There may be defined or chosen a reference coordinate system, which is desirably that of the rendering and used for representing rays. Then, data coordinates may be converted into the reference ones. So, each data voxel may be located in the reference coordinate system. The conversion does not need to be done beforehand. It may be done on the fly during rendering. In summary, for processing voxel-represented data, it is not necessary to harmonize data resolution beforehand. Instead, each ray may be sampled, and the data value may be calculated at the sampled point by interpolation, on the fly during the rendering.

At least one of the optical determination steps, the fluorescence determination step and the rendering step, may be implemented or may use at least one first artificial-intelligence-based (AI-based) algorithm and/or (pre-trained) machine learning algorithm. Accordingly, any one of the steps of the method of the present disclosure may be carried out by a machine-learning-based or any other AI-algorithm.

At least one second artificial-intelligence-based algorithm and/or a pre-trained machine learning algorithm may carry out a predefined task as a function of the rendered data representation. For example, said task may comprise at least one of the following non-limiting lists: a regression task, a classification task, and a segmentation task, or any other pre-defined task.

Accordingly, the data representation of the present disclosure is not necessarily a visual data representation but may be any kind of data set which can be processed by an (AI-based) algorithm, for example one of the tasks mentioned above.

Beside this, the data representation may also comprise or consist of any parameters determined in the rendering step. For example, such parameters may include the distance between a predetermined point of an intervention device (for example the tip of a biopsy needle) and a predetermined region of interest of the medium (which is for instance selected by the user and/or determined by the data of a modality and/or source, for instance by SWE data, and/or using an Artificial Intelligence dedicated module).

Moreover, the rendered data representation may be used as input for an AI-based algorithm (i.e. the second artificial-intelligence-based algorithm and/or a pre-trained machine learning algorithm). Said AI-based algorithm may then determine any information which may support the user in understanding or interpreting the original data (i.e. the multi-modality and/or multi-source data).

The present disclosure further relates to a computer program comprising computer-readable instructions which when executed by a data processing system cause the data processing system to carry out the method according to the present disclosure.

The present disclosure further relates to a system (i.e. a processing system) for processing multi-modality and/or multi-source data of a volumetric medium. Said system may comprise a processing unit configured to:

- determine for at least one of a plurality of volume units of the medium an optical property based on the data of at least a first modality and/or source,
- determine for at least one of the volume units a fluorescence property based on the data of at least a second modality and/or source, and
- render a data representation of the volumetric medium based on the determined optical and fluorescence properties of the volume units.

The system may be or may comprise a medical system, for example an ultrasound imaging system.

The present disclosure may also relate to a recording medium readable by a computer and having recorded thereon a computer program including instructions for executing the steps of the method according to the present disclosure, when said program is executed by a computer.

The disclosure and it's embodiments may be used in the context of medical devices dedicated to human beings, animals, but also any material to be considered such as metallic pieces, gravel, pebbles, etc..

It is intended that combinations of the above-described elements and those within the specification may be made, except where otherwise contradictory.

It is to be understood that both the foregoing general description and the following detailed description are exemplary and explanatory only, are provided for illustration purposes and are not restrictive of the disclosure, as claimed.

The accompanying drawings, which are incorporated in and constitute a part of this specification, are provided for illustration purposes and illustrate embodiments of the disclosure and together with the description and serve to support and illustrate the principles thereof.

BRIEF DESCRIPTION OF THE DRAWINGS

Fig. 1 shows a flowchart of a method for processing multi-modality and/or multi-source data of a volumetric medium according to embodiments of the present disclosure;
Fig. 2 shows a first exemplary embodiment of the method of fig. 1;
Fig. 3 shows a second exemplary embodiment of the method of fig. 1;
Fig. 4a shows a schematic illustration of a ray-tracing method according the present disclosure; and
Fig. 4b shows a schematic image obtained by the ray-tracing method of fig. 4a.

DESCRIPTION OF THE EMBODIMENTS

Reference will now be made in detail to exemplary embodiments of the disclosure, examples of which are illustrated in the accompanying drawings. Wherever possible, the same reference numbers will be used throughout the drawings to refer to the same or like parts.

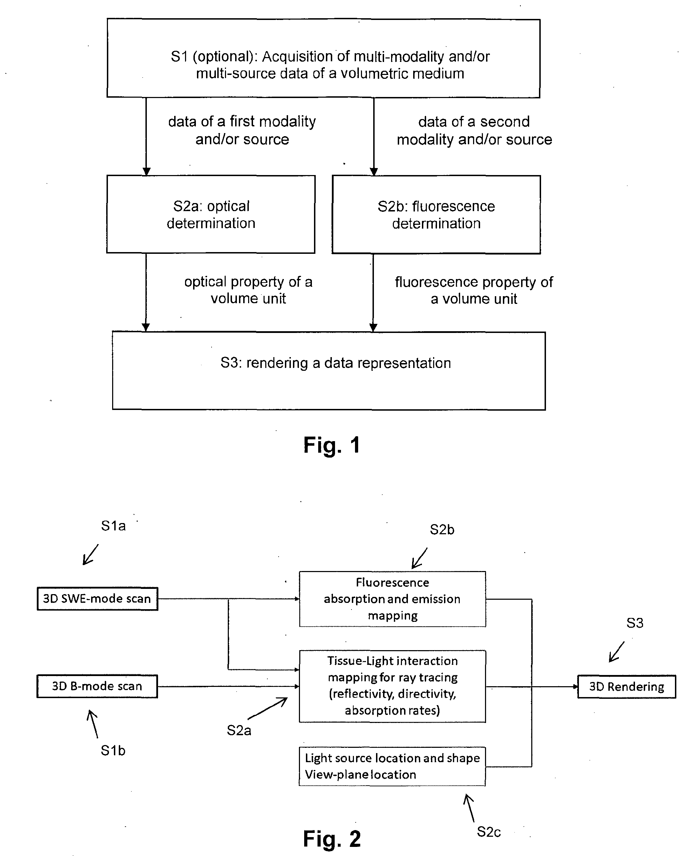

Fig. 1 shows a flowchart of a method for processing multi-modality and/or multi-source data of a volumetric medium according to embodiments of the present disclosure.

In an optional acquisition step S1 data of different modalities and/or different sources of a volumetric medium are acquired. In one example, the volumetric (i.e. 3D) medium may be a living tissue and in particular human tissues of a patient or an animal or a plant.

In an optical determination step S2a for at least one of a plurality of volume units of the medium an optical property is determined based on the data of at least a first modality and/or source. In other words, said step receives as input data of at least a first modality and/or source (or for example of the first and second modalities and/or sources) of step S1 and processes them to determine an optical property (for example a light absorption rate) of a volume unit.

In a fluorescence determination step S2b for at least one of the volume units a fluorescence property is determined based on the data of at least a second modality and/or source. In other words, said step receives as input data of at least a second modality and/or source of step S1 and processes them to determine a fluorescent property (for example a luminosity and/or color) of a volume unit.

In a rendering step S3 a data representation of the volumetric medium is rendered based on the determined optical and fluorescence properties of the volume units. In other words, said step receives as input the determined fluorescence and/or optical properties for each volume unit for which a determination has been made in steps S2a and S2b and processes them to render a data representation.

For example, the data representation may comprise or may be a visual representation or may be in the form of one or several images, for example a two-dimensional (2D) or a three-dimensional (3D) image. The data representation may also comprise a visual or graphical representation with a plurality of depth levels. For example, it may comprise a set of 2D images of different depth levels of the medium. The data representation may further comprise a holograms or augmented reality scenes or any other scene which allows a user to navigate in the scene. However, the data representation of the present disclosure is not necessarily a visual data representation but may be any kind of data set which can be processed by for example an (AI-based) algorithm, for example one of the tasks mentioned above.

The rendering step may use a conventional rendering technique, for example maximum intensity projection or ray tracing. However, in the method of the present disclosure an additional step of fluorescence modelling is added, for example to the conventional ray-tracing framework. This allows the different data to numerically emit personalized fluorescence colors, then to be ray-traced through the optical model, and finally to be presented in a unified scene.

A further example of a rendering technique applicable in the present disclosure is multiplanar reformation (MPR). MPR is an image processing technique, which extracts two-dimensional (2D) slices from a 3D volume using arbitrarily positioned orthogonal or oblique planes. Another possible example is surface rendering (SR) what requires the object surface to be explicitly or implicitly modelled. SR may advantageously be used to render an intervention device in the medium. The surface is then shaded and rendered, for example by the method of ray-tracing.

Still another example is volume rendering (VR) which is in particular useful for the rendering step of the present disclosure which displays the entire 3D data as a 2D image without computing any intermediate geometrical representation.

The method of the present disclosure implies several advantages:

For example, a unified way of visualizing multiple 3D imaging modes (for example, 3D B-mode and 3D SWE) is provided. Furthermore, the user experience of visualizing multi-mode 3D data can be improved. In particular, the intuitive interpretation of ultrasound volumes, with functional information such as SWE. It is also capable of in-volume navigation with customizable perspective. It can further be integrated in Augmented Reality devices for visual guidance, for instance using virtual glasses or the like. It also facilitates education or training of professionals and non-professionals, as well as their communication between peers. Moreover, information exploration with free viewpoint and reducing constraints on imposed way of imaging becomes possible. Qualitative visual monitoring capability may thus be provided, for example for surgical or therapeutical usage such as ablation.

The method may further be implementable with conventional hardware such as a GPU (i.e. no additional hardware device is required). Moreover, the technique enables multi-mode 3D image blending without object or surface segmentation. The computational cost is independent of number of objects, and therefore it is not required to make multiple renderings for multiple objects. The computational cost only depends on number of volume units, and is compatible to current computational capacity. The coloring relies on physics principle of fluorescence and not on arbitrary shading, even if it can of course be customised, depending of the habits and preferences of the user. This facilitates practical parameters optimization by providing more physical intuition.

The method may be carried out by a processing system. Such a system may comprise a processing unit, and optionally an acquisition system (for example a transducer) and/or a visualisation system (for example a display, glasses).

It is though possible that the acquisition system and/or the visualisation system is external to processing system. For example, a transducer (or a group of transducers) may be remotely connectable to the processing system. In one exemplary embodiment the transducer is an IOT device and/or is connectable to an IOT device and/or to a smartphone forming the processing unit and/or visualization system. The transducer may be connectable to the processing system via the internet, the 'cloud', 4G or 5G protocols, WIFI, any local network or any other data contact or remote connection.

It is further possible that the processing system and the visualisation system are remotely connectable, for example via the internet, the 'cloud', 4G or 5G protocols, WIFI, any local network or any other data contact or remote connection.

The processing unit may comprise for example a central processing unit (CPU) and/or a graphical processing unit (GPU) communicating with optional buffer memories, optionally a memory (MEM) linked to the central processing system; and optionally a digital signal processor (DSP) linked to the central processing system.

The optional acquisition system may comprise at least one transducer, for example a single transducer configured to transmit a pulse and receive the medium (i.e. tissue) response. Also, it is possible to use a plurality of transducers and/or a transducer array. The array may be is adapted to perform a bidimensional (2D) imaging of the medium, but the array could also be a bidimensional array adapted to perform a 3D imaging of the medium. The transducer array may also be a convex array including a plurality of transducers aligned along a curved line. The same transducer(s) may be used to transmit a pulse and receive the response, or different transducers may be used for transmission and reception.

The optional visualization system may comprise one or several displays, virtual reality lenses, or any other electronic device for sowing 2D or 3D images, holograms or augmented reality scenes.

For example, using augmented reality lenses may permit a user to virtually look into the inner of medium by placing the visual representation of the medium at the actual position of the medium. This may in particular be useful, in case an intervention device is inserted into the medium and its movement inside towards a point of interest (for example a lesion) shall be controlled.

Moreover, using augmented reality lenses may permit a surgeon or any other doctor or user to navigate through the visual representation of the medium (for example changing the point of view and/or the viewing angle) with free hands, meaning that he/she may at the same time carry out any interventions (for example using an intervention device according to the present disclosure). For this purpose, the movement of the augmented reality lenses may be sensed, and the corresponding sensor signals may control the point of view and/or the viewing angle of the visual presentation.

It is also possible to present the data representation (in particular a visual representation) on different devices such as displays and/or augmented reality lenses. Such visualization devices may also be combined depending on the need and/or their usability in specific cases (for instance depending on the looking direction of the user during an examination or intervention, the resolution of a visual representation, etc.).

The processing system may comprise or may be connectable to a device for ultrasound imaging. The transducers may thus be ultrasound transducers. However, the processing system may comprise or may be connectable to any imaging device or medical system using other waves than ultrasound waves (waves having a wavelength different than an ultrasound wavelength).

Accordingly, the multi-modality and/or multi-source data may origin from different sources, for example an ultrasound system and another system, for example a medical imaging system like computer tomography, magnetic resonance or positron emission tomography or a tracking system like an electromagnetic (EM) tracker.

According to a further embodiment, the modality and/or source data may comprise intervention device information. Said intervention device information may for example origin from a system for intervention device imaging and/or tracking, as described below. Said intervention device information may indicate the position and/or orientation of an intervention device (for example its 6D-pose) placed in the medium. Said intervention device information may optionally comprise predetermined information of the shape of the intervention device. The position and/or orientation may be defined by providing respective coordinates for the intervention device in a coordinate system of the scanned medium, as described above. The coordinate system may also predefine the structure of the volume units.

An example of different data modalities may be data acquired by a system (for example one medical system) in different acquisition modes, for example brightness mode (B-mode), shear wave elastography (SWE) and strain elastography in case of ultrasound imaging.

Fig. 2 shows a first exemplary embodiment of the method of fig. 1. In other words, the method of fig. 2 corresponds to that one of fig. 1 unless described differently. In particular, the multi-modality and/or multi-source data acquired (i.e. scanned) in optional step 1 may be of two different modalities, ie. (3D) B-mode as a first modality acquired (cf. Sib) and (3D) SWE as a second modality acquired (cf. S1a).

In an optical determination step S2a for a plurality of volume units (for example voxels) of the medium (i.e. tissue) an optical property is determined based on the data of the first modality of step S1b and of the second modality of step S1a.

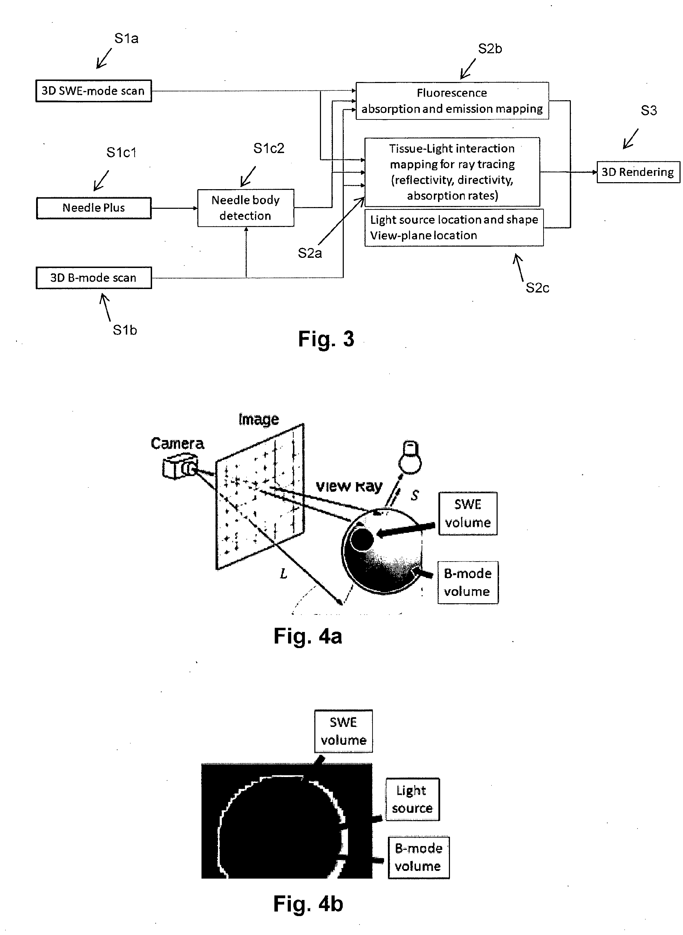

In particular, the optical property is determined according to a tissue-light interaction mapping applied to the data of the first and second modalities and/or source of the volume units. The tissue-light interaction mapping optionally determines at least one of a reflectivity, directivity and absorption rates. The determined optical properties of the volume units may be suitable to render an image in step S3 according to a ray-tracing technique (cf. fig. 4a and 4b in this regard).

In a fluorescence determination step S2b for a plurality of volume units of the medium a fluorescence property is determined based on the data of the second modality of step S1a. In other words, said step receives SWE data as input and processes them to determine a fluorescent property (for example a luminosity and/or color) of a volume unit. As a consequence, an area in the medium (for example a lesion) detected by the SWE scan may be marked by a certain fluorescent property. The fluorescence property may be determined according to a light emission and/or absorption mapping applied to the data of the second modality.

Moreover, in a step S2C, a virtual light source may be defined and positioned in a predefined (geometric) position and or pose with regard to the medium. Said light source may illuminate the rendered scene showing the medium according to the ray-tracing technique in step S3. For this purpose, a 2D view plane may be defined and positioned in a predefined (geometric) position and/or pose with regard to the medium.

In a rendering step S3 a data representation of the volumetric medium is rendered based on the determined optical and fluorescence properties of the volume units of steps S2a and S2b and the illumination characteristics and view plane defined in step S2C. The rendering step may use a ray-tracing technique as further described in context of fig. 4a and 4b.

Fig. 3 shows a second exemplary embodiment of the method of fig. 1. The method of fig. 3 corresponds to that one of fig. 2 unless described differently.

In particular, in addition to the method of fig. 2 steps S1c1 and S1c2 have been added, in which intervention device information, for example from a biopsy needle, is acquired. Said intervention device information may indicate the position and/or orientation of an intervention device (for example its 6D-pose) placed in the medium. Said intervention device information may further comprise predetermined information of the shape of the intervention device.

Exemplary intervention devices comprise a needle, in particular a biopsy needle, a marker, a catheter, a protheses, a micromechanical system (MEMS) MEMS, a stent, a valve, a bypass, a pacemaker, or any other device which may be or has been inserted into the medium. Note that the intervention devices according to the present disclosure are not limited to surgical intervention but to any intervention where an external device is inserted and/or implanted into the medium (for instance a marker.). The intervention device may thus also be any external device.

In one exemplary embodiment, said intervention device information may be acquired by a system for tracking and/or imaging an intervention device (representing for example a fourth modality and/or source according to the present disclosure). Said system may be an ultrasound imaging system, for example the same one which is used for obtaining SWE data and/or B-mode data. Alternatively, it may be an external system or may comprise further means (for example, sensors, transducers or marker), as described below.

For example, an intervention device in the form of needle may be used that is configured to emit signals from its tip. By capturing the signal of the needle tip, it is possible to localize the tip. Hence, this tip point may be rendered inside the scene of the data representation showing the medium. This type of visualization can advantageously provide valuable information of biopsy location in real time to a doctor.

In step S1C1 (in fig. 3 referred to as a mode "needle plus") the position and/or orientation of the intervention device in the medium (or in relation to the medium, in case it is outside the medium) may be detected or tracked by scanning the intervention device using first scanning waves adapted as a function of predetermined information of the intervention device. The predetermined information of the intervention device may comprise for example pre-determined wave reflection and absorption characteristics. Accordingly, the scanning waves may be adapted to more reliably detect the intervention device, in particular comprising its position and orientation in a surrounding medium. In this case, the intervention device may be regarded as a passive device,

The scan may be done by a probe associated to the processing system and optionally of the ultrasound system used for B-mode scans and/or SWE data. Alternatively, the probe may use other than ultrasound waves.

According to a further alternative, the intervention device may be equipped with means configured to enable detecting its position and/or orientation. In this case, the intervention device may be regarded as an active device. For example, the intervention device may be equipped with a sensor, marker and/or a transducer for measuring and/or signalling the needle/marker position. In a further example, the system for tracking and/or imaging an intervention device may comprise an electromagnetic (EM) tracker. The EM tracker may generate an EM field in which EM micro-sensors of the intervention device are tracked.

In step S1C2 a (3D) region in the medium is determined which is occupied by the intervention device, i.e. where the intervention device is located. For this purpose, the position and/or orientation of the intervention device detected in step S1C1 is used. Moreover information regarding the (3D) shape of the intervention device is used. The information of the shape of the intervention device may comprise 3D surface information of the intervention device. It may also comprise a set of images (for example 2D or 3D) from different perspectives of the intervention device.

Said information may be (pre)determined in prior scanning step (for example carried out before the optical determination step and/or the fluorescence determination step). As shown in the exemplary embodiment of fig. 3, the B-mode data acquired in step S1b may be used to determine the shape. However, the shape may also determined in another scanning method.

Alternatively, the shape of the intervention device may be obtained from a data storage, for example a local data storage of the processing system or a remote data storage, for example a cloud system. In one exemplary utilisation, the shape may be predetermined once and then stored, such that it can be obtained at any time afterwards.

However, it is noted that the intervention device may in some cases evolve during the time and/or even during intervention itself. For example, it may be deformed, reduced in size or be twisted during the intervention by mechanical force. The intervention may also emit radiation or incorporated fluid (for instance radioactive one), in case it is for example a marker. It is therefore desirable that the system is configured to render the intervention device based on prestored data and additionally based on "live data", which are obtained during the intervention. For example, such "live data" may consist of B-mode data acquired in step Sib, as described above.

Moreover, the data storage may store intervention device information of different intervention devices. The information of one intervention device may not only comprise its shape but also further parameters of it, for example technical characteristics (such as its material, its spatial dimensions, its weight) and/or manufacturer information (such as a product ID, a manufacturer ID).

For example, the B-mode data or any other scanning data acquired in the scanning step may be used to determine a product ID of the intervention device. Based on this product ID, the correct information regarding the (3D) shape of the intervention device may be obtained from the data storage.

As a result, a 3D model of the intervention device is obtained and set into a geometrical relation to the medium.

In the fluorescence determination step S2b for at least one of the volume units an (intervention-device-specific) fluorescence property may be determined based on the intervention device information. This determination may be done by identifying all volume units which are occupied by the intervention device according to its position and/or orientation and its shape. In particular, all these volume units may be colored according to the fluorescence properties determined for the detected intervention device.

As a consequence, an area in the medium occupied by the intervention device may be marked by a certain fluorescent property which is different to that one used for the SWE data of step S1a. Moreover, the intervention device may be visualized in a respective 3D-model comprising the medium and the intervention device.

According to a further exemplary embodiment, in the rendering step S2c at least a portion of the data representation may be rendered based on intervention device information. Said intervention device information may indicate the position and/or orientation of an intervention device placed in the medium and predetermined information of the shape of the intervention device. Accordingly, instead of determining fluorescence properties for volume units and rendering afterwards, it is also possible to use the intervention device information directly for rendering the final data representation. As a consequence, it is not necessary to render the whole scene (i.e. the complete data representation), for instance each time the intervention device moves, but only the intervention device, allowing a more efficient real time update of the scene.

For example, a surface rendering (SR) method may be used to render the intervention device. In this case the fluorescence property may be applied to the surface of the intervention device, and the SR rendering is run together with for example B-mode data of step S1b. So it becomes possible to simultaneously deal with data with explicit surface information of the intervention device and data without segmentation information from b-mode data.

As a consequence of using the intervention device information in the fluorescence determination step and/or the rendering step, it becomes possible to visualize both an intervention device (for example a biopsy needle) and the medium (for example anatomical structures).

Furthermore, it is possible to determine where the intervention device is predicted to move to in the medium (for example its moving direction and/or a moving path). Said prediction may be made by a respectively trained (AI-based) algorithm.

For example, the intervention device may comprise a biopsy gun and a biopsy needle to be moved to a region of interest in the medium.

Moreover, a trained AI-based algorithm may predict the type and/or shape of an intervention device based on data obtained in steps S1c1 and/or S1c2. Based on said prediction an image of the intervention device may be rendered in the rendering step at the predicted location of the intervention device in the medium. The image may be generated based on predetermined image data of the intervention device stored in a local data storage of the processing system or remotely stored, for example in a cloud system.

In one embodiment, the rendered data representation may be stored stored and/or presented at different phases of the intervention, during intervention and/or after an examination of a patient (optionally including an intervention). It may also be updated at every examination. For example, the data representation of a previous examination and a present examination may be presented (visually and/or in the form of parameters), in order to allow a verification of any evolution, for example of a tumour or any other lesion. This may also apply to any potential evolution of an implanted device. For example, the device may comprise one or several markers or any other element which is inserted into the medium for a longer time period, for instance a time period with at least two examinations. In such a case, the evolution of said implanted device may be monitored, for instance the movement and/or deformation of a marker in a visual presentation or in the form of numbers (for example movement distance between two or more examinations).

Fig. 4a shows a schematic illustration of a ray-tracing method according the present disclosure. The ray-tracing rendering method (for example used in a method as described in context of fig. 1 to 3) may have the following sub-steps:

1. From B-mode data of step 1b (cf. fig. 1 to 3), in step 2a an attenuation mapping is calculated by mapping B-mode data to an attenuation coefficient at every volume unit, for example at every voxel, of the B-mode image. In a computationally efficient alternative, B-mode data are mapped to an attenuation coefficient (only) at the volume units along a ray which is then used for the ray tracing method described below. In other words, the mapping may be "ray-by-ray". "Concerned" means those volume units for which data in the respective modality and/or source (here B-mode) are provided.

2. From the light source data of step S2c, a ray-tracing method is applied in step 3 to derive incident light intensity at each concerned volume unit by considering light attenuation, and optionally the directivity of incident ray w.r.t the local gradient.

3. For the SWE volume of step 1a, a user may customize the fluorescence color by defining in for step 2b a 3x3 absorption matrix M. This matrix encodes, for each of RGB fluorescence color component, the photon absorption rates as a function of incident RGB intensities. RGB is merely an example of possible color coding. A possible alternative of RGB would be for example HSV (hue, saturation, value).

4. For the SWE volume, the user may also customize a 3x3 fluorescence emission matrix E in step 2b. On the main diagonal of E, the emission rate is provided for each of the RGB components.

5. Then, the incident light intensity at each concerned volume unit x, i.e., $[I_R(\mathbf{x})\, I_G(\mathbf{x})\, I_B(\mathbf{x})]^T$ is converted to fluorescence light in the SWE volume:

$$[F_R(\mathbf{x})\, F_G(\mathbf{x})\, F_B(\mathbf{x})]^T = E \cdot M \cdot [I_R(\mathbf{x})\ I_G(\mathbf{x})\ I_B(\mathbf{x})]^T \qquad (1)$$

where:

$F_R(\mathbf{x})$ is the red component of the fluorescence light emitted by the volume unit x,
$F_G(\mathbf{x})$ is the green component the of fluorescence light emitted by the volume unit x,
$F_B(\mathbf{x})$ is the blue component of the fluorescence light emitted by the volume unit x,
E is the 3x3 emission matrix recording the fluorescence emission rates,
M is the 3x3 absorption matrix recording the light absorption rates,
$I_R(\mathbf{x})$ is the red component of the incident light at the volume unit x,
$I(\mathbf{x})$ is the green component of the incident light at the volume unit x,
$I_B(\mathbf{x})$ is the blue component of the incident light at the volume unit x,

6. Along the viewing ray L crossing a given pixel to render in the data representation, its RGB intensity components $[V_R\ V_G\ V_B]^T$ are computed in step S3 by combining light-intensity from the incident light source arrived inside the B-mode volume units met by the ray L, and the fluorescence intensity from the fluorescence emission.

7. These two parts of intensities may again be modulated by conventional ray-tracing method in step S3 along the view ray L by applying attenuation along this ray. The attenuation factors for the two parts of intensity contribution can be different and may be customizable.

Fig. 4b shows a schematic image obtained by the ray-tracing method of fig. 4a. Fig. 4b shows an exemplary scenario of a rendered data representation. In this example the representation is based on two ultrasound modalities, for example one from 3D B-mode to show normal tissue, and one from 3D SWE mode to highlight pathological tissue (i.e. a lesion) with a user-defined or predefined fluorescence color.

An example of rendering of a volume containing a synthetic ball (considered as B-mode volume), and a second volume containing a small ball (considered as SWE volume) of different tissue elasticity is shown.

The gray values in the B-mode volume may be considered proportional to the light attenuation factors. Accordingly, since the virtual light source is defined to be positioned behind the ball and rather on the right side, a region on the left side of the ball appears darker due to an increased attenuation. An upper small region represents blended SWE information, for example a region of interest, defined by the user or an AI module or detected via the SWE scan. Said region representing blended SWE information may have a predefined or user-defined fluorescent color.

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially" and/or "approximately" and/or "generally" should be understood to mean falling within such accepted tolerances.

Although the present disclosure herein has been described with reference to particular embodiments, it is to be understood that these embodiments are merely illustrative of the principles and applications of the present disclosure.

It is intended that the specification and examples be considered as exemplary only, with a true scope of the disclosure being indicated by the following claims.

**Claims**

1. A method for processing multi-modality and/or multi-source data of a medium, the method comprising the following steps:

an optical determination step in which for at least one of a plurality of volume units of the medium an optical property is determined based on the data of a first modality and/or source,
a fluorescence determination step in which for at least one of the volume units a fluorescence property is determined based on the data of a second modality and/or source, and

a rendering step in which a data representation of the medium is rendered based on the determined optical and fluorescence properties of the volume units.

2.  The method according to claim 1, wherein
    the data representation of the volumetric medium comprises a visual representation and/or a 2D or 3D image.

3.  The method according to any one of the preceding claims, wherein
    the optical property comprises at least a light absorption rate of a volume unit, and/or
    the optical property is determined according to a first medium-light interaction mapping applied to the data of the first modality and/or source of the at least one volume unit, and
    the first medium-light interaction mapping optionally determines at least one of a reflectivity, directivity and absorption rate as a function of the data of the first modality and/or source.

4.  The method according to any one of the preceding claims, wherein
    the fluorescence property is determined according to a first light emission and/or absorption mapping applied to the data of the second modality and/or source of the at least one volume unit.

5.  The method according to any one of the preceding claims, wherein
    in the optical determination step an optical property is determined for at least one of the volume units based on the data of a third modality and/or source and according to a second medium-light interaction mapping being different to the first medium-light interaction mapping, and/or
    in the fluorescence determination step a fluorescence property is determined for at least one of the volume units based on the data of a fourth modality and/or source and according to a second light emission and/or absorption mapping being different to the first light emission and/or absorption mapping.

6.  The method according to any one of the preceding claims, wherein
    the fluorescence determination step and the optical determination step are carried out simultaneously and/or in parallel.

7.  The method according to any one of the preceding claims, wherein
    the determined optical and fluorescence properties are stored in a data storage and the rendering step is carried out afterwards one or several times based on the stored properties, or
    the rendering step is carried out simultaneously with the fluorescence determination step and/or the optical determination step.

8.  The method according to any one of the preceding claims, wherein
    the data of the first modality and/or source comprise reflectivity information from B-mode ultrasound imaging, and/or
    the data of the second modality and/or source comprise tissue elasticity information from shear wave elastography ultrasound imaging.

9.  The method according to any one of the preceding claims, wherein
    the data of the second modality and/or source comprise intervention device information indicating the position and/or orientation of an intervention device placed in relation to the medium and/or predetermined information of the shape of the intervention device, and
    in the fluorescence determination step for at least one volume unit a fluorescence property is determined based on the intervention device information by identifying those volume units which are occupied by the intervention device.

10. The method according to any one of the preceding claims 1 to 8, wherein in the rendering step at least a portion of the data representation is rendered based on intervention device information indicating the position and/or orientation of an intervention device placed in the medium and predetermined information of the shape of the intervention device.

11. The method according to any one of the preceding claims, wherein
    the rendering step comprises a ray-tracing rendering step in which the data representation is rendered according to a ray tracing volume rendering method.

12. The method according to the preceding claim, wherein
    the ray-tracing rendering step comprises at least one of the sub-steps of:

- positioning a virtual light source in a predefined geometric relation to the medium,
- defining a depth of field and/or an aperture shape,
- positioning a virtual view plane in a predefined geometric relation to the medium, wherein the data representation is rendered as a function of the virtual view plane.

13. The method according to any one of the preceding claims, wherein the multi-modality and/or multi-source data comprise image data and/or 3D data of the medium, and/or volume unit comprises at least one voxel.

14. The method according to any one of the preceding claims, wherein at least one of the optical determination step, the fluorescence determination step and the rendering step, are implemented or use at least one first artificial-intelligence-based algorithm and/or machine learning algorithm.

15. The method according to any one of the preceding claims, wherein at least one second artificial-intelligence-based algorithm and/or a pre-trained machine learning algorithm carries out a predefined task as a function of the rendered data representation, said task optionally comprising at least one of: a regression task, a classification task, and a segmentation task.

16. A computer program comprising computer-readable instructions which when executed by a data processing system cause the data processing system to carry out the method according to any one of preceding method claims.

17. A system for processing multi-modality and/or multi-source data of a medium, comprising a processing unit configured to:

    determine for at least one of a plurality of volume units of the medium an optical property based on the data of a first modality and/or source,
    determine for at least one of the volume units a fluorescence property based on the data of a second modality and/or source, and
    render a data representation of the medium based on the determined optical and fluorescence properties of the volume units.

**Fig. 1**

**Fig. 2**

S1a

3D SWE-mode scan

S1c1

Needle Plus

S1c2

Needle body
detection

3D B-mode scan

S1b

S2b

Fluorescence
absorption and emission mapping

S3

Tissue-Light interaction
mapping for ray tracing
(reflectivity, directivity,
absorption rates)

3D Rendering

S2a

Light source location and shape
View-plane location

S2c

**Fig. 3**

Camera

Image

view Ray

S

L

SWE
volume

B-mode
volume

**Fig. 4a**

SWE
volume

Light
source

B-mode
volume

**Fig. 4b**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YOUK JI HYUN ET AL: "Three-dimensional shear-wave elastography for differentiating benign and malignant breast lesions: comparison with two-dimensional shear-wave elastography", EUROPEAN RADIOLOGY, vol. 23, no. 6, 1 June 2013 (2013-06-01), pages 1519-1527, XP055840078, DE ISSN: 0938-7994, DOI: 10.1007/s00330-012-2736-3 * abstract * * | 1-16 | INV.<br>G01S15/89<br>G01S7/52<br>A61B8/08 |
| A | VAN SLOUN RUUD J G ET AL: "Deep Learning in Ultrasound Imaging", PROCEEDINGS OF THE IEEE, IEEE. NEW YORK, US, vol. 108, no. 1, 1 January 2020 (2020-01-01), pages 11-29, XP011763165, ISSN: 0018-9219, DOI: 10.1109/JPROC.2019.2932116 [retrieved on 2019-12-27] * the whole document * | 14,15 | |
| X | US 2015/150535 A1 (FAN LIEXIANG [US] ET AL) 4 June 2015 (2015-06-04) * abstract *; figures 1, 3 * * paragraph [0003] - paragraph [0009] * * paragraph [0015] - paragraph [0030] * * paragraph [0060] - paragraph [0081] * | 1,16,17 | TECHNICAL FIELDS SEARCHED (IPC)<br><br>G01S<br>A61B |

The present search report has been drawn up for all claims

| Place of search | Date of completion of the search | Examiner |
|---|---|---|
| Munich | 14 September 2021 | Zaneboni, Thomas |

EPO FORM 1503 03.82 (P04C01)

# EP 4 060 383 A1

## ANNEX TO THE EUROPEAN SEARCH REPORT
## ON EUROPEAN PATENT APPLICATION NO.

EP 21 31 5044

This annex lists the patent family members relating to the patent documents cited in the above-mentioned European search report.
The members are as contained in the European Patent Office EDP file on
The European Patent Office is in no way liable for these particulars which are merely given for the purpose of information.

14-09-2021

| Patent document cited in search report | | Publication date | Patent family member(s) | | Publication date |
|---|---|---|---|---|---|
| US 2015150535 | A1 | 04-06-2015 | CN | 104688266 A | 10-06-2015 |
| | | | EP | 2889004 A1 | 01-07-2015 |
| | | | KR | 20150065158 A | 12-06-2015 |
| | | | US | 2015150535 A1 | 04-06-2015 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ZHANG Q ; EAGLESON R ; PETERS TM.** Volume visualization: a technical overview with a focus on medical applications. *J Digit Imaging.,* August 2011, vol. 24 (4), 640-64